(19) **Europäisches Patentamt / European Patent Office / Office européen des brevets**

(11) **EP 1 832 293 A1**

(12) **EUROPEAN PATENT APPLICATION**
published in accordance with Art. 158(3) EPC

(43) Date of publication:
**12.09.2007 Bulletin 2007/37**

(51) Int Cl.:
*A61K 33/24* (2006.01)    *A61K 31/663* (2006.01)
*A61P 19/00* (2006.01)    *A61P 35/04* (2006.01)
*A61P 43/00* (2006.01)

(21) Application number: **05814661.4**

(22) Date of filing: **06.12.2005**

(86) International application number:
**PCT/JP2005/022327**

(87) International publication number:
**WO 2006/062072 (15.06.2006 Gazette 2006/24)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **10.12.2004 JP 2004358471**

(71) Applicant: **TMRC Co., Ltd.**
**Tokyo 107-0052 (JP)**

(72) Inventor: **EKIMOTO, Hisao**
**1150042 (JP)**

(74) Representative: **Jones, Helen M.M. et al**
**Gill Jennings & Every LLP**
**Broadgate House**
**7 Eldon Street**
**London EC2M 7LH (GB)**

(54) **REMEDY FOR CANCER METASTASIS AND CANCER METASTASIS INHIBITOR**

(57) To provide a therapeutic agent for metastatic cancer and a cancer metastasis inhibitor which are highly safe and efficaciously treat metastatic cancer and inhibit cancer metastasis, and especially efficaciously treat and inhibit cancer metastasis to bone and liver.

There are provided a therapeutic agent for metastatic cancer and a cancer metastasis inhibitor each of which includes a platinum complex having an anticancer activity as an active ingredient. The platinum complex is prefer- ably cisplatin. The therapeutic agent for metastatic cancer is advantageously usable for treating metastatic cancer to bone and/or liver. The cancer metastasis inhibitor is advantageously usable as a cancer metastasis inhibitor for inhibiting metastasis to bone and/or liver. These agents can advantageously further include a bisphosphonate as another active ingredient.

EP 1 832 293 A1

**Description**

Technical Field

The present invention relates to therapeutic agents for metastatic cancer and metastasis inhibitors. Specifically, it relates to therapeutic agents for metastatic cancer and metastasis inhibitors which are efficacious for treating and inhibiting cancer metastasis to bone and/or liver.

Background Art

Various therapies such as surgery (surgical operation), radiotherapy, chemotherapy, immunotherapy, and combinations of these are conducted for treating cancer, and a variety of anticancer agents and cartinostatics have been developed. In addition, with a quantum leap in surgical techniques, the cure rate of primary cancer has been markedly increased. However, cancer cells, if being surgically-unresectable, may undergo metastasis to another organ. Even if cancer seems to be completely resected and cured when observed by the naked eye, a trace amount of cancer cells may remain and undergo metastasis to another organ. It is difficult to prevent these metastases into other organs, and metastases are observed with a considerably high rate in cancer patients.

Cancer metastases into the bone and liver accompanied with advance of cancer in cancer patients are currently mainly treated by adjunctive therapies with antitumor drugs such as 5-FU, cisplatin, and epiadriamycin. In particular, unresectable colorectal cancer metastases to liver are treated, for example, by general chemotherapy with fluorouracil (5-FU) alone or in combination with another anticancer agent, intrahepatic administration of floxuridine (FUDR), or intrahepatic administration of FUDR in combination with another agent (refer to Non-Patent Document 1).

Bisphosphonate preparations have recently known as therapeutic agents for bone lesion accompanied with metastasis of malignant tumor to bone. In addition, Non-Patent Document 2 reports the metastasis inhibitory effect of prophylactic administration of bisphosphonate preparations against metastases typically of breast cancer and prostatic cancer to bone. Non-Patent Document 3, however, reports that bisphosphonate preparations do not contribute to life prolongation of patients and that these preparations have weak or no growth-inhibitory action against cancer cells in the bone.

Non-Patent Document 1: T. G. Allen-Mersh et al.: Quality of Life and Survival with Continuous Hepatic-Artery Floxuridine Infusion for Colorectal Liver Metastases.: "Lancet": 1994, 344: p. 1255-1260

Non-Patent Document 2: G. N. Hortobagyi et al.: Efficacy of Pamidronate in Reducing Skeletal Complications in Patients with Breast Cancer and Lytic Bone Metastasis. "New England Journal of Medicine": 1996, 335: p. 1785-1791

Non-Patent Document 3: P. F. Conte et al.: Delay in Progression of Bone Metastases in Breast Cancer Patients Treated with Intravenous Pamidronate: Results from a Multinational Randomized Controlled Trial. "Journal of Clinical Oncology": 1996, 14: p. 2552-2559

Disclosure of Invention

Problems to be Solved by the Invention

For completely curing cancer, it is important not only to cure cancer in a primary site but also control cancer in a site of metastasis. Particularly, regarding typically to breast cancer, prostatic cancer, lung cancer, and gastrointestinal cancer, both primary cancer and metastatic cancer metastasized to bone and liver are resistant to cure. Consequently, it is important to treat bone and liver metastatic cancer and to inhibit the metastases, and demands have been made to improve therapeutic and inhibitory effects of drugs against metastatic cancers to bone and liver.

Accordingly, an object of the present invention is to provide a therapeutic agent for metastatic cancer and a metastasis inhibitor which efficaciously treat metastatic cancer and inhibit cancer metastasis, especially efficaciously treat and inhibit bone and liver metastatic cancer, and which are highly safe.

Means for Solving the Problems

After intensive investigations to solve the problems, the present inventors have found that a platinum preparation has a therapeutic activity against metastatic cancer and also has an inhibitory activity against cancer metastasis. Such a platinum preparation is known as an anticancer agent for inhibiting the growth of cancer cells as a result of binding with their DNAs. The present invention has been made based on these findings.

Specifically, a therapeutic agent for metastatic cancer and a metastasis inhibitor according to the present invention each include a platinum complex having an anticancer activity as an active ingredient.

Cisplatin is advantageously used as the platinum complex. Primary cancer in the therapeutic agent for metastatic cancer and cancer to be inhibited from metastasis in the metastasis inhibitor include, for example, colorectal cancer, prostatic cancer, gynecological cancer, gastric cancer, multiple myeloma, liver cancer (hepatic cancer), lung cancer, pancreatic cancer, thyroid cancer, kidney cancer, biliary canal cancer, gallbladder cancer, neuroblastoma, and Hodgkin lymphoma. The term "gynecological cancer" means and includes breast cancer, corpus uteri cancer, ovarian cancer, and cervical cancer.

The therapeutic agent for metastatic cancer according to the present invention can be advantageously used for treating metastatic cancer metastasized to bone and/or liver. The metastasis inhibitor according to the present invention can be advantageously used as a metastasis inhibitor for inhibiting metastasis to bone and/or liver. The therapeutic agent for metastatic cancer and the metastasis inhibitor according to the present invention may advantageously further contain a bisphosphonate as another active ingredient. This treats and inhibits metastasis especially to bone. These agents can also be used as orally-bioavailable agents.

Advantages

The therapeutic agent for metastatic cancer and metastasis inhibitor according to the present invention, which contain a platinum complex as an active ingredient, can treat and/or inhibit cancer metastases, especialy cancer metastases to bone and liver. By further containing a bisphosphonate, they can further efficaciously treat and/or inhibit the metastases. Such a platinum preparation inhibits not only metastasis but also growth of cancer, and the resulting agent can be an agent that can concurrently efficaciously treat, inhibit, and prevent metastases. In addition, they can also be used as orally-bioavailable agents and can be provided as satisfactorily safe therapeutic agents and inhibitors for cancer metastases. Best Mode for Carrying Out the Invention

The present invention provides a therapeutic agent for metastatic cancer and a metastasis inhibitor (antimetastatic agent) each containing a platinum complex as an active ingredient. Such platinum complexes have been known to be combined with DNA and to thereby inhibit growth of cancer cells. Platinum complexes for use in the present invention are not specifically limited, and examples thereof include cisplatin, carboplatin, nedaplatin, and oxaliplatin, of which cisplatin is advantageously used.

Primary cancer in the therapeutic agent for metastatic cancer according to the present invention and cancer to be inhibited from metastasis in the metastasis inhibitor according to the present invention include, for example, colorectal cancer, prostatic cancer, gynecological cancer, gastric cancer, multiple myeloma, liver cancer, lung cancer, pancreatic cancer, thyroid cancer, kidney cancer, biliary canal cancer, gallbladder cancer, neuroblastoma, and Hodgkin lymphoma. The agents are especially useful for breast cancer, one of gynecological cancer, as well as prostatic cancer, lung cancer, and colorectal cancer, because these cancers often undergo metastases typically to bone and liver and are resistant to cure.

A therapeutic agent for metastatic cancer and a metastasis inhibitor according to the present invention can be formulated into pharmaceutical compositions containing an active ingredient and pharmacologically acceptable pharmaceutical aids and can be administered orally or non-orally. The dosage forms of these agents are not specifically limited. When they are orally administered, they can be formulated into solid preparations such as tablets, powders, or capsules, or liquid preparations such as syrups according to an ordinary procedure. When they are non-orally administered, they can be formulated into common dosage forms such as injections or inhalants.

Pharmaceutical compositions relating to the present invention can be prepared by adding pharmacologically and pharmaceutically acceptable additives according to necessity to active ingredients. Examples of the additives include known additives such as excipients, binders, diluents, disintegrators, stabilizers, and lubricants. When the compositions are formulated into injections, they must be subjected to proper sterilization.

When a therapeutic agent for metastatic cancer and a metastasis inhibitor according to the present invention are orally administered, these agents are preferably formulated into capsules each including a composition essentially containing a platinum complex and a polyethylene glycol. Such a platinum complex itself has carcinogenicity and shows strong adverse reaction. When formulated into the capsules, however, the resulting capsules are preparations that can be satisfactorily absorbed and causes less adverse reaction. The water content of the composition contained in the capsules should be 10 percent by W/W or less relative to the essential components (refer to Japanese Unexamined Patent Application Publication No. 10-279478).

The dose of a platinum complex relating to the present invention varies depending typically on the type of carcinoma, symptom, age, sexuality, and body weight of the patient, the type and dosage form of the platinum complex, and how the platinum complex is administered. Upon oral administration, the platinum complex may be administered at a dose in terms of the amount of active ingredient of 0.01 to 0.5 mg, and preferably 0.02 to 0.2 mg per 1 kg of body weight per adult per day, once a day for consecutive three weeks, followed by drug withdrawal for one week. This administration course is appropriately repeated at least six times and at most twelve times. Upon administration by intravenous injection or drip infusion, the administration is appropriately carried out at a dose of 0.01 to 0.4 mg, and preferably 0.02 to 0.1 mg per 1 kg of body weight per adult per one day, once a day for consecutive days.

A therapeutic agent for metastatic cancer and a metastasis inhibitor according to the present invention are preferably each further contain a bisphosphonate as an active ingredient. The "bisphosphonate" for use in the present invention refers to a compound having a P-C-P (phosphorus-carbon-phosphorus) structure as a basic structure and having an activity on the bone, such as osteoclastic bone resorption inhibitory activity. Examples of bisphosphonates include etidronate, clodronate, tiludronate, pamidronate, alendronate, incadronate, zoledronate, minodronate, risedronate, ibandronate, and mixtures of these. The dose, administration method, dosing period, and other parameters of a bisphosphonate are properly selected according typically to the type of cancer, symptom, age, sexuality, and body weight of the patient, and the type of the bisphosphonate.

Preparation methods of these bisphosphonates can be found in U. S. Patent No. 3468935 for etidronate; J. Org. Chem. 32. 4111. 1967 for clodronate; EP Patent No. 100718 for tiludronate; U. S. Patent No. 4327039 for pamidronate; U. S. Patent No. 4705651 for alendronate; EP Patent No. 325482 for incadronate; U. S. Patent No. 4939130 for zoledronate; EP Patent No. 186405 for risedronate; and U. S. Patent No. 4927814 for ibandronate.

EXAMPLES

The present invention will be illustrated in further detail with reference to several examples below, which, however, are never intended to limit the scope of the present invention.

EXPERIMENTAL EXAMPLE 1

Therapeutic and Inhibitory Effects on Cancer Metastasized from Murine Colorectal Cancer Colon-26 to Liver

When cancer cells are transplanted into the spleen of mice, metastatic cancer is selectively formed in the liver. Use of this model enables various therapeutic experiments in order to control metastasis to liver (refer to MORIKAWA, Kiyoshi, Nude Mouse Spleen Transplantation Method as Human Cancer Liver Metastasis Model, "Oncologia": 1989, 22 (2): p. 100-102, and L. Kopper et al.: Experimental Model for Liver Metastasis Formation Using Lewis Lung Tumor.: "Journal of Cancer Research Clinical Oncology": 1982, 103: p31-38).

Specifically, a liver metastasis model of murine colorectal cancer Colon 26 was established using 6-week old CDF1 mice. The therapeutic and inhibitory activities were assayed using, as an index, the ratio (life prolongation rate; T/C percentage) of the survival time of a drug-administered group (10 mice per group) to the survival time of a non-drug-administered (control) group (10 mice per group). In the drug-administered group, cisplatin as a platinum complex was dissolved in physiological saline and was orally administered. In the control group, physiological saline alone was orally administered. One day after the cancer cell transplantation, the administration was started, and cisplatin was administered at a dose of 1 mg per kg per day with drug-administered group. The results are shown in Table 1 below.

[Table 1]

|  | Control Group | Cisplatin-administered Group |
|---|---|---|
| Median Survival Time (day) | 16.0 | 31.0 |
| T/C(%) | 100 | 194 |

Dissection of died mice revealed that each of them died of bleeding of liver infected with cancer and that no metastasis to another organ was observed. These results demonstrate that cisplatin shows a remarkable life prolongation effect and has therapeutic and inhibitory effects against liver metastasis.

EXPERIMENTAL EXAMPLE 2

Therapeutic and Inhibitory Effects Against Metastasis in Animal Model of Metastasis to Bone

The therapeutic effect and inhibitory effect against metastasis in an animal model of metastasis to bone were evaluated in the following manner. Initially, a test animal was anaesthetized with thiamylal, the precordia was disinfected with iodine and 70% ethanol and was incised to 1 cm along with the midline to expose the ribs. The second interspace of the left sternum was needled to a depth of 2 mm with a needle of a tuberculin syringe 30 G. After observing that fresh blood came up into the syringe, cultured tumor cells in an amount of $10^4$ cells in 0.1 mL were injected over one minute or longer. At the time when the animal (mouse) was weakened or paralyzed, the mouse was killed with ether anesthesia, and evaluation was conducted (refer to F. Arguello et al.: A Murine Model of Experimental Metastasis to Bone and Bone Marrow. "Cancer Research": 1988, 48: 6876-6881).

Melanoma B16 cells ($10^4$/0.1 mL) were transplanted to the left ventricle of 8-week old female C57BL/6 mice (12 mice per group). Starting one day after the transplantation, cisplatin was orally administered at a dose of 1 mg per kg per day for consecutive days. At the time three weeks after the transplantation when the mice were weakened or paralyzed, the mice were killed under inhalation with ether, the target organs were fixed with 10% formalin, and evaluation of tumor metastasis was conducted. The number of individual mice where metastasis was observed is shown in Table 2 below.

[Table 2]

| | | Control Group | Cisplatin |
|---|---|---|---|
| Vertebral Column | Cervical Region | 0/10 | 0/10 |
| | Pectoral Region | 9/10 | 6/10 |
| | Lumbar Region | 8/10 | 4/10 |
| | Sacral Spine | 6/10 | 1/10 |
| Pectoral Region | Rib | 9/10 | 5/10 |
| | Breast Bone | 4/10 | 2/10 |
| | Blade Bone | 7/10 | 5/10 |

Table 2 demonstrates that cisplatin significantly inhibits metastasis to bone and inhibits growth of cancer cells in the bone.

EXPERIMENTAL EXAMPLE 3

Therapeutic and Inhibitory Effects of Combination Use with Bisphosphonate Against Metastasis in Animal Model of Metastasis to Bone

The therapeutic and inhibitory effects of combination use with a bisphosphonate against metastasis in an animal model of metastasis to bone were evaluated in the following manner. Human multiple myeloma U266 cells in an amount of $2 \times 10^6$ in 0.2 ml were transplanted to NOD/SCID/$\gamma$c$^{null}$ (NOG) mice through their tail vein (10 mice per group). Starting one day after the tumor transplantation, cisplatin was orally administered at a dose of 1 mg per kg per day for consecutive 28 days (cisplatin group). 21 days after the transplantation, Aredia containing pamidronate, one of bisphosphonates, as an active ingredient was orally administered at a dose of 1 mg per kg per day for consecutive 8 days (Aredia group). In addition, cisplatin at a dose of 1 mg per kg per day and Aredia at a dose of 1 mg per kg per day were administered in combination according to the same schedules as the single administration, respectively (cisplatin plus Aredia group). 29 days after the tumor transplantation, the human IgE (hIgE) level in the serum was measured, and the therapeutic effect against metastasis to bone was determined (refer to Y. Miyakawa et al.: Establishment of a new model of human multiple myeloma using NOD/SCID/$\gamma$cnull (NOG) mice. "Biochemical and Biophysical Research Communication": 2004, 313: 258-262). The data of human IgE levels in the sera are shown in Table 3 below.

[Table 3]

| | Number of Animal | Dosing Schedule | Dosing Period | hIgE Level |
|---|---|---|---|---|
| Control Group (no treatment) | 9 | — | — | 351.7ng/mL |
| Cisplatin Group | 9 | 1 day later to 28 days later | 28 days | 210.5ng/mL |
| Aredia Group | 9 | 21 day later to 28 days later | 8 days | 297.7ng/mL |
| Cisplatin plus Aredia Group | 9 | Cisplatin: 1 day later to 28 days later, Aredia: 21 days later to 28 days later | Cisplatin: 28 days Aredia: 8 days | 165.2ng/mL |

Table 3 demonstrates that cisplatin alone or in combination with Aredia inhibits the increase in hIgE level due to metastasis to bone and inhibits the growth of cancer cells in the bone.

EXPERIMENTAL EXAMPLE 4

Effect and Nephrotoxicity of Low-dose Daily Administration of Cisplatin to Nude Mice with Human Lung Cancer

The effect and nephrotoxicity of low-dose daily intravenous administration of cisplatin were determined and compared with those of intermittent intravenous administration. Human large cell lung carcinoma LC-1 cells were transplanted to 6-week old female BALB/cA-nu/nu mice subcutaneously in the dorsolateral region, and cisplatin was administered from the point of time when the tumor volume reached 100 to 200 mm$^3$. In the low-dose daily intravenous administration, cisplatin was administered at a dose of 1.33 mg per kg per a course for consecutive 5 days, and this course was repeated a total of three times to a total dose of 20 mg per kg (low-dose daily administered group). In the intermittent intravenous administration, cisplatin was administered at a dose of 6.67 mg per kg a total of three times every 7 days to a total dose of 20 mg per kg (intermittently administered group). As an index of the effect, the tumor growth delay percentage (%) was determined by calculation according to the following equation:

$$\text{Tumor Growth Delay Percentage (\%)} = (A-B)/B \times 100 \qquad (1)$$

wherein "A" represents the number of days required for the tumor to increase in its volume as much as 8 times in each cisplatin-administered group; and "B" represents the number of days required for the tumor to increase in its volume as much as 8 times in the control group. In addition, blood urea nitrogen (BUN) was measured as an index of nephrotoxicity. The results are shown in Table 4 below.

[Table 4]

|  | Tumor Growth Delay Percentage (%) | BUN Level (mg/dL) |
|---|---|---|
| Intermittently Administered Group | 265 | 45.5 |
| Low-dose Daily Administered Group | >293 | 20.4 |

Table 4 demonstrates that low-dose daily administration of cisplatin inhibits growth of cancer without exhibiting nephrotoxicity.

**Claims**

1.  A therapeutic agent for metastatic cancer, comprising a platinum complex having an anticancer activity as an active ingredient.

2.  The therapeutic agent for metastatic cancer according to claim 1, wherein the platinum complex is cisplatin.

3.  The therapeutic agent for metastatic cancer according to claim 1, wherein the metastatic cancer is metastasized from a primary cancer selected from the group consisting of colorectal cancer, prostatic cancer, gynecological cancer, gastric cancer, multiple myeloma, liver cancer, lung cancer, pancreatic cancer, thyroid cancer, kidney cancer, bile duct cancer, gallbladder cancer, neuroblastoma, and Hodgkin lymphoma.

4.  The therapeutic agent for metastatic cancer according to claim 1, for treating metastatic cancer metastasized to bone and/or liver.

5.  The therapeutic agent for metastatic cancer according to claim 4, further comprising a bisphosphonate as another active ingredient.

6.  The therapeutic agent for metastatic cancer according to claim 1, as an orally-bioavailable agent.

7.  A cancer metastasis inhibitor comprising a platinum complex having an anticancer activity as an active ingredient.

8.  The metastasis inhibitor according to claim 7, wherein the platinum complex is cisplatin.

9.  The metastasis inhibitor according to claim 7, wherein the cancer to be inhibited from metastasis is selected from the group consisting of colorectal cancer, prostatic cancer, gynecological cancer, gastric cancer, multiple myeloma, liver cancer, lung cancer, pancreatic cancer, thyroid cancer, kidney cancer, bile duct cancer, gallbladder cancer, neuroblastoma, and Hodgkin lymphoma.

10. The metastasis inhibitor according to claim 7, as an inhibitor for inhibiting metastasis to bone and/or liver.

11. The metastasis inhibitor according to claim 10, further comprising a bisphosphonate as another active ingredient.

12. The metastasis inhibitor according to claim 7, as an orally-bioavailable agent.

<table>
<tr><td colspan="2"><b>INTERNATIONAL SEARCH REPORT</b></td><td>International application No.<br>PCT/JP2005/022327</td></tr>
</table>

A.  CLASSIFICATION OF SUBJECT MATTER
*A61K33/24*(2006.01), *A61K31/663*(2006.01), *A61P19/00*(2006.01), *A61P35/04* (2006.01), *A61P43/00*(2006.01)

According to International Patent Classification (IPC) or to both national classification and IPC

B.  FIELDS SEARCHED

Minimum documentation searched (classification system followed by classification symbols)
A61K33/24, A61K31/663, A61P19/00, A61P35/04, A61P43/00

Documentation searched other than minimum documentation to the extent that such documents are included in the fields searched
| | | |
|---|---|---|
| Jitsuyo Shinan Koho | 1922-1996 | Jitsuyo Shinan Toroku Koho 1996-2006 |
| Kokai Jitsuyo Shinan Koho | 1971-2006 | Toroku Jitsuyo Shinan Koho 1994-2006 |

Electronic data base consulted during the international search (name of data base and, where practicable, search terms used)
CAOLD(STN), CAplus(STN), MEDLINE(STN), REGISTRY(STN), JMEDPlus(JOIS), JST7580(JOIS), JSTPlus(JOIS)

C.  DOCUMENTS CONSIDERED TO BE RELEVANT

| Category* | Citation of document, with indication, where appropriate, of the relevant passages | Relevant to claim No. |
|---|---|---|
| X | JP 02-502182 A  (HENKEL KGAA), 19 July, 1990 (19.07.90), Page 11, lower left column to page 12, lower right column; page 5, lower right column & WO 88/06149 A1    & EP 345294 A1 & US 5034552 A     & US 5256653 A & EP 345294 B1 | 1-12 |
| X<br>Y | KLENNER, T., Anticancer-agent-linked phosphonates with antiosteolytic and antineoplastic properties: a promising perspective in the treatment of bone-related malignancies? Journal of cancer research and clinical oncology, 1990, Vol.116, No.4, pp.341-50, page 341, left column, lines 14 to 18 | 1,3-5,7,9-11<br>2,6,8,12 |

| ☒ Further documents are listed in the continuation of Box C. | ☐ See patent family annex. |
|---|---|

* Special categories of cited documents:
"A" document defining the general state of the art which is not considered   to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search<br>19 January, 2006 (19.01.06) | Date of mailing of the international search report<br>31 January, 2006 (31.01.06) |
|---|---|
| Name and mailing address of the ISA/<br>Japanese Patent Office | Authorized officer |
| Facsimile No. | Telephone No. |

Form PCT/ISA/210 (second sheet) (April 2005)

**INTERNATIONAL SEARCH REPORT**

| International application No. |
| --- |
| PCT/JP2005/022327 |

C (Continuation). DOCUMENTS CONSIDERED TO BE RELEVANT

| Category* | Citation of document, with indication, where appropriate, of the relevant passages | Relevant to claim No. |
| --- | --- | --- |
| X<br>Y | JP 62-005911 A (SLOAN KETTERING INST CANCER),<br>12 January, 1987 (12.01.87),<br>Page 4, upper left column; page 5, lower left<br>column<br>& EP 201804 A1     & US 4686104 A<br>& US 4704277 A     & EP 201804 B1 | 1-4,6-10,12<br>5,11 |
| X<br>Y | JP 11-322616 A (BAE I),<br>24 November, 1999 (24.11.99),<br>Page 9, Table 9<br>& EP 955052 A1     & DE 19831579 A1<br>& JP 3007627 B2     & CN 1235124 A<br>& KR 99084594 A     & KR 272835 B<br>& US 6309672 B1     & US 2002/028253 A1<br>& US 6589567 B2     & US 2004/018246 A1<br>& EP 955052 B1     & ES 2230662 T3<br>& CA 2298093 A1     & CA 2298093 C<br>& MX 2000001237 A1 | 1-3,7-9<br>4-6,10-12 |
| X<br>Y | WO 2001/97849 A1 (MITSUBISHI PHARM CO., LTD.),<br>27 December, 2001 (27.12.01),<br>Pages 23, 51 to 58<br>& AU 200174598 A     & EP 1295607 A1<br>& KR 2003011106 A     & US 2003/165576 A1<br>& CN 1447697 A     & US 6930115 B2 | 1-3,7-9<br>4-6,10-12 |
| X<br>Y | JP 2003-515534 A (ONCOZYME PHARMA INC.),<br>07 May, 2003 (07.05.03),<br>Page 28<br>& WO 2001/35935 A2     & AU 200113765 A<br>& EP 1231910 A2     & CA 2483352 A1<br>& CA 2388674 C     & AU 780538 B2 | 1-3,7-9<br>4-6,10-12 |
| X | GROHN P., Successful management of a<br>widespread osteosarcoma. A case report,<br>Medical principles and practice :<br>international journal of the Kuwait<br>University, Health Science Centre, 2004<br>Jan-Feb, Vol.13, No.1, pages 54 to 56, page 54,<br>right column, lines 1 to 7; page 55, right<br>column, lines 1 to 8; page 56, left column,<br>lines 4 to 20 | 1-12 |
| X | JP 03-504715 A (HENKEL KGAA),<br>17 October, 1991 (17.10.91),<br>& DE 3804686 A     & WO 89/07453 A1<br>& EP 401243 A1     & EP 401243 B1<br>Page 6, lower left column to lower right column;<br>page 4, upper left column; page 7, upper right<br>column | 1-12 |
| P,X | WO 2005/000858 A2 (ODANI, Akira),<br>06 January, 2005 (06.01.05),<br>(Family: none) | 1-12 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10279478 A
- US 3468935 A
- EP 100718 A
- US 4327039 A
- US 4705651 A
- EP 325482 A
- US 4939130 A
- EP 186405 A
- US 4927814 A


**Non-patent literature cited in the description**

- **T. G. ALLEN-MERSH et al.** Quality of Life and Survival with Continuous Hepatic-Artery Floxuridine Infusion for Colorectal Liver Metastases. *Lancet,* 1994, vol. 344, 1255-1260
- **G. N. HORTOBAGYI et al.** Efficacy of Pamidronate in Reducing Skeletal Complications in Patients with Breast Cancer and Lytic Bone Metastasis. *New England Journal of Medicine,* 1996, vol. 335, 1785-1791
- **P. F. CONTE et al.** Delay in Progression of Bone Metastases in Breast Cancer Patients Treated with Intravenous Pamidronate: Results from a Multinational Randomized Controlled Trial. *Journal of Clinical Oncology,* 1996, vol. 14, 2552-2559
- *J. Org. Chem.,* 1967, vol. 32, 4111
- **MORIKAWA, KIYOSHI.** Nude Mouse Spleen Transplantation Method as Human Cancer Liver Metastasis Model. *Oncologia,* 1989, vol. 22 (2), 100-102
- **L. KOPPER et al.** Experimental Model for Liver Metastasis Formation Using Lewis Lung Tumor. *Journal of Cancer Research Clinical Oncology,* 1982, vol. 103, 31-38
- **F. ARGUELLO et al.** A Murine Model of Experimental Metastasis to Bone and Bone Marrow. *Cancer Research,* 1988, vol. 48, 6876-6881
- **Y. MIYAKAWA et al.** Establishment of a new model of human multiple myeloma using NOD/SCID/γcnull (NOG) mice. *Biochemical and Biophysical Research Communication,* 2004, vol. 313, 258-262